# EUROPÄISCHE PATENTANMELDUNG

(11) **EP 0 523 471 A1**
(43) Veröffentlichungstag der Anmeldung: **20.01.1993**
(21) Anmeldenummer: 92111292.6
(22) Anmeldetag: 03.07.1992
(51) Int. Cl.: B29C 53/80, B29C 53/64

(54) **Verfahren zur Herstellung von Faserverbund-Bauteilen**

(30) Priorität: 19.07.1991 DE 4124015
(71) Anmelder: MAN Ceramics GmbH, 94453 Deggendorf (DE)
(72) Erfinder: Dierl, Rudolf, W-8060 Dachau (DE)

(57) **Zusammenfassung**

Zur Herstellung von Bauteilen mit mindestens einer Lage aus unidirektionalen Fasern wird ein Verfahren vorgeschlagen, bei dem ein vorgefertigter Stützkern (10) zusammen mit den unidirektionalen Fasern (25) und den aus einer Flechtmaschine (14, 17) kommenden Flechtfasern (21, 22) durch ein Fadenauge (11) gezogen wird, wobei die zumindest teilweise außen zu liegen kommenden Flechtfasern die unidirektionalen Fasern an den Stützkern andrücken. Mit diesem Verfahren können sowohl Bauteile mit regelmäßiger Geometrie als auch mit sich veränderndem Querschnitt, sowie Bauteile mit gebogenen Konturen mit längs des Bauteiles gerichteten Fasern bestückt werden.

## Beschreibung

Die Erfindung bezieht sich auf ein Verfahren zur Herstellung von Bauteilen mit mindestens einer Lage aus längs des Bauteiles gerichteten unidirektionalen Fasern, bei dem ein Stützkern hergestellt wird, der anschließend zusammen mit das Bauteil zumindest teilweise umgebenden, unter Spannung gehaltenen unidirektionalen Fasern durch eine Flechtmaschine geführt wird, wobei die unidirektionalen Fasern sowie ein Flechtwerk auf das Bauteil aufgelegt und mittels Harz verklebt werden (nach Patent 40 04 473).

Aus der DE 34 13 601 A1 ist ein Verfahren bekannt, bei dem zur Herstellung von Verbundprofilen ein vorgefertigter Stützkern zusammen mit ihn umgebenden Verstärkungsfasern in einen Formkanal eingeführt, mit flüssigem Kunstharz getränkt und anschließend einem Aushärtungsprozeß unterworfen wird. Hierdurch können Bauteile mit längs des Bauteiles gerichteten, sogenannten unidirektionalen Fasern fertigungstechnisch einfach umgeben werden. Allerdings ist das Verfahren nur bei Bauteilen geeignet, die einen konstanten Querschnitt haben.

Dieser Mangel wird gemäß einem Vorschlag nach der DE 39 19 508 dadurch behoben, daß anstelle der starren Form eine Membran verwendet wird, deren Öffnung sich automatisch an die Kontur des durchzuführenden Bauteiles anpaßt.

Bei Anwendung dieser Methode können sich jedoch noch Probleme mit gekrümmten Bauteilen ergeben. Die den Kern umgebenden, unter Spannung gehaltenen Fasern werden bei Krümmungen des Stützkernes den kürzeren Weg einnehmen wollen, wodurch ein Verrutschen der Faser zur konkaven Seite des Bauteiles sowie das Abheben der Fasern an konkaven Seiten möglich ist.

Um Bauteile jeder Konfiguration unter Beibehaltung der fertigungtechnischen Einfachheit mit unidirektionalen Fasern (UD-Fasern) überziehen zu können, wird gemäß dem Hauptpatent das bekannte Verfahren dahingehend abgewandelt, daß Flechtfasern zum Andrücken der UD-Fasern verwendet werden.

Die Fasern des Flechtwerkes winden sich automatisch um den Kern herum und drücken dabei zwischen Kern und Flechtfasern befindliche unidirektionalen Fasern an den Kern Hierbei ersetzen die Flechtfasern nicht nur die bei dem bekannten Verfahren verwendete starre Form bzw. Membran, sondern sie bilden gleichzeitig einen Torsionskasten für das Bauteil, der die bei Belastung auftretenden Radialkräfte aufnimmt.

Die gleichzeitige Aufbringung von UD- und Flechtfasern ist beispielsweise auch aus der US-PS 3,592,884 bekannt. Hierbei handelt es sich jedoch um zylindrische Bauteile, wobei das Bauteil durch ein als Fadenauge für die UD-Fasern ausgebildetes Rohr durchgezogen, mit UD-Fasern belegt und am Rohrausgang mit dem Flechtwerk umgeben wird. Dieses bekannte Verfahren ist nur für Bauteile mit regelmäßiger Kontur anwendbar.

Gemäß dem Hauptpatent wird Abhilfe dadurch geschaffen, daß zumindest ein Teil der unidirektionalen Fasern in das Flechtwerk einbezogen wird, wodurch deren Führung entlang der Mantellinie des Bauteiles durch die Flechtfasern erzwungen wird. Diese Ausgestaltung eignet sich insbesondere für stark gekrümmte Bauteile, bei denen eine möglichst genaue Führung der unidirektionalen Fasern entlang der Mantellinien gewünscht wird.

In diesem Fall werden die gewählten unidirektionalen Fasern mit Flechtfasern unterlegt, so daß diese Fasern in ihrer Wirkung an Festigkeit einbüßen. Ein Ausgleich kann allerdings durch Anhebung der Faserlagenzahl erreicht werden. Es ist aber auch möglich, durch Wahl sehr dünner Flechtfasern die Festigkeitseinbußen zu verringern. Es ist selbstverständlich jede Kombination möglich, bei der mehr oder weniger unidirektionale Fasern zwischen dem Kern und den Flechtfasern bzw. innerhalb des Geflechtes geführt werden.

Der Erfindung liegt nun die Aufgabe zugrunde, das Verfahren der eingangs genannten Art dahingehend weiterzuentwickeln, daß eine höhere Flexibilität in der Führung der UD-Fasern möglich ist.

Die Aufgabe wird erfindungsgemäß durch die Maßnahme des Anspruchs 1 gelöst.

Durch die Führung des Bauteiles durch die Flechtmaschine in der Form, daß die Achse des Bauteiles im Flechtbereich parallel auf der Flechtmaschinenachse zu liegen kommt, werden unregelmäßige Oberflächen des Bauteiles im Flechtbereich annähernd achsensymmetrisch orientiert. In Verbindung mit der höheren Fadenspannung der Flechtfasern wird das Anlegen der Flechtfasern und insbesondere der UD-Fasern in der gewünschten Orientierung sichergestellt.

Bei Bauteilen mit starken Krümmungen wird ferner vorgeschlagen, den Winkel der Flechtfasern gegenüber der Bauteil- bzw. Flechtmaschineflachse durch Veränderung der relativen Geschwindigkeit zwischen Maschine und Bauteil zu variieren, und zwar derart, daß die Flechtfasern die UD-Fasern sowohl in Bereichen mit annähernd konstantem Querschnitt als auch in Bereichen mit abnehmendem oder zunehmendem Querschnitt des Bauteiles sicher andrücken. Der Winkel, den die Flechtfaser zur Flechtmaschinenachse bildet, wird in erster Linie durch die gewünschte Faserlage auf dem Bauteil gewählt. Wenn dieser Winkel relativ klein, z.B. 30 oder 40 Grad sein soll, wird der Winkel bei abnehmendem Bauteilquerschnitt allmählich erhöht, bis der Winkel in der Talsohle fast 90 Grad erreicht, um bei anschließender Zunahme des Querschnitts wieder langsam, unter Umständen bis auf den gewünschten Wert verkleinert zu werden. Um also ein Abrutschen der Fasern zu vermeiden und die UD-Fasern sicher anzudrücken, werden die Flechtfasern bei geometrisch kritischen Bereichen des Bauteiles mehr oder weniger eine Orientierung in Umfangsrichtung des Bauteiles einnehmen.

Die Veränderung des Flechtfaserwinkels erfolgt durch Veränderung der Abzugsgeschwindigkeit, d. h. der Geschwindigkeit, mit der entweder das Bauteil durch ein Fadenauge oder der Flechtring bewegt wird.

Das Verfahren eignet sich zur serienmäßigen Herstellung von Bauteilen in einem Arbeitsgang, wobei die Bauteile jeweils aus einem gesamten langen Strang abgetrennt wurden, der mit einem Kern hergestellt ist.

Bei Bauteilen, die zumindest an einem Ende ohne Kern, d. h. ausschließlich aus UD-Fasern und Flechtfasern bestehen sollen, sind getrennte Stützkerne zu verwenden, die entweder untereinander mit wenigen Fasern verbunden sind oder die während eines kontinuierlichen Flechtvorganges maschinell einzeln in das Flechtauge eingeschoben werden.

Durch Kombination der vorstehend beschriebenen Maßnahmen mit einer Umflechtung einzelner UD-Fasern kann das Verfahren in Bezug auf die Parameter flexibler ausgelegt werden, indem eine Maßnahme die andere stützt und damit z. B. die Flechtfaserorientierung besser der gewünschten Orientierung angepaßt wird.

Die Einbringung eines Harzes erfolgt nach konventionellen Methoden, z.B. durch Tränken der Fasern vor oder nach deren Aufbringung auf den Stützkern. Das nach dem oben beschriebenen Verfahren hergestellte Bauteil wird anschließend oder während des Durchziehens durch das Fadenauge einer Wärmebehandlung unterzogen, um das Harz auszuhärten.

Die Erfindung erstreckt sich auf die Anwendung des erfindungsgemäßen Verfahrens zur Herstellung von Knochenimplantaten, insbesondere zur Herstellung des Schaftes für eine Hüftgelenk-Endoprothese. Ein vorgefertigter Kern wird vorzugsweise vollständig mit straffgezogenen, unidirektionalen Fasern umlegt, wobei die Flechtfasern die unidirektionalen Fasern umgeben.

Zur Anwendung des erfindungsgemäßen Verfahrens kann eine im Handel erhältliche Flechtmaschine verwendet werden, die um Faserführungen für die unidirektionalen Fasern erweitert wird.

Die Erfindung wird anhand von in der Zeichnung schematisch dargestellten Ausführungsbeispielen näher beschrieben.

Es zeigen:
- Fig. 1 und 2: eine Anlage zum Durchführen des Verfahrens im Schnitt bzw. in Draufsicht,
- Fig. 3: den Faserverlauf an einem Bauteil,
- Fig. 4 und 5: je einen Querschnitt eines fertigen Bauteiles und
- Fig. 6: den Flechtprozeß mit zwei Flechtmomenten.

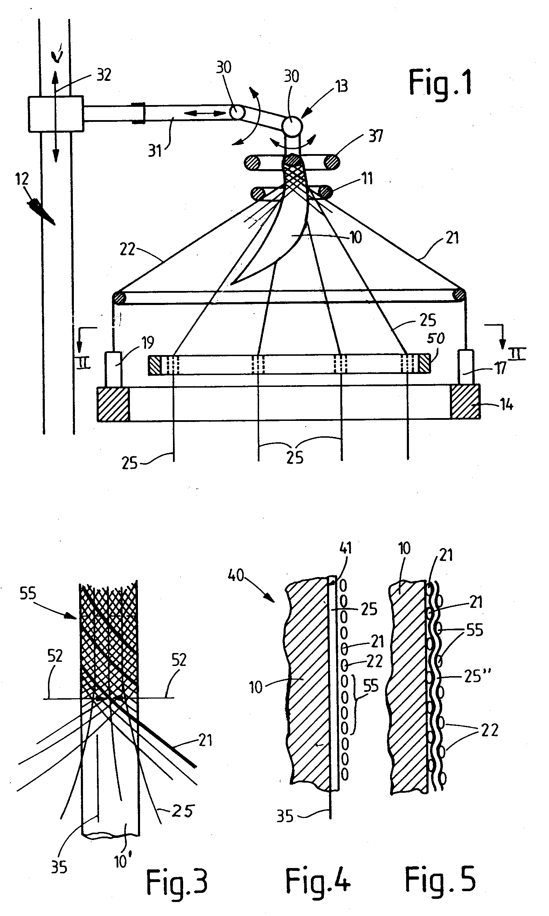

In, Fig. 1 ist die Herstellung eines Prothesenschaftes gezeigt, der zunächst aus einem vorgefertigten Stützkern 10 besteht. Der Stützkern 10 wird durch ein Fadenauge 11 einer Flechtmaschine 12 mittels eines Greifers 13 durchgezogen. Die Flechtmaschine 12 besteht aus einem Klöppel-Führungsring 14, auf dem zwei sinusförmige Führungsschienen 15 bzw. 16 (Fig. 2) vorgesehen sind. In der einen Schiene 15 wird eine bestimmte Anzahl von Klöppel 17 einer ersten Gruppe in eine Richtung 18 geführt, während eine zweite Gruppe Klöppel 19 auf der zweiten Schiene 16 in die Gegenrichtung 20 geführt wird.

Bei der so beschriebenen Bewegung der Klöppel 17 bzw. 19 wird jede einzelne Faser 21 einmal unter eine andere Faser 22 und einmal über eine nächste Faser 22 gelegt. Zusätzlich zu den Flechtfasern 21,22 der beiden Klöppelgruppen 17, 19 sind über einen inneren Ring 50 (Fig. 2) geführte unidirektionale Fasern 25 vorgesehen, die zwischen dem Kern 10 und den Flechtfasern 21,22 durch das Fadenauge 11 durchgezogen werden. Sämtliche Faserenden werden mit dem Greifer 13 verbunden, der gleichzeitig den Stützkern 10 trägt. Mit einer vorgegebenen, variablen Abzugsgeschwindigkeit v wird der Greifer 13 vom Fadenauge 11 wegbewegt. Für gekrümmte oder unregelmäßige Bauteile, wie es beim Prothesenschaft der Fall ist, ist der Greifer 13 mit Gelenken 30, Teleskopeinrichtung 31 oder dergleichen ausgerüstet, um zusätzlich zur Abzugsbewegung 32 auch noch Schwenkbewegungen und radiale Verschiebungen durchführen zu können. Damit kann das Bauteil jeweils so gerichtet werden, daß die Achse 60 des Bauteilbereiches e₁, das gerade umflochten wird, auf der Flechtmaschinenachse 61 liegt. Auf diese Weise werden die unter Spannung gehaltenen unidirektionalen Fasern 25 stets den Mantellinien 35 (Fig. 3) des Bauteiles folgen.

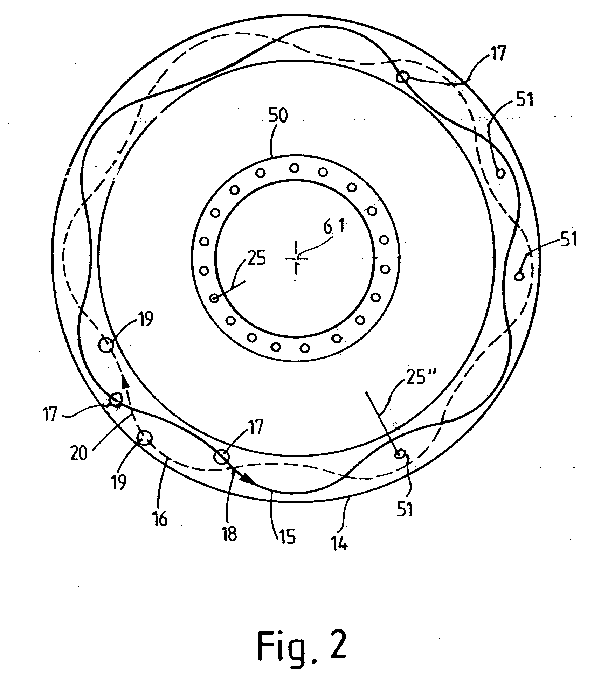

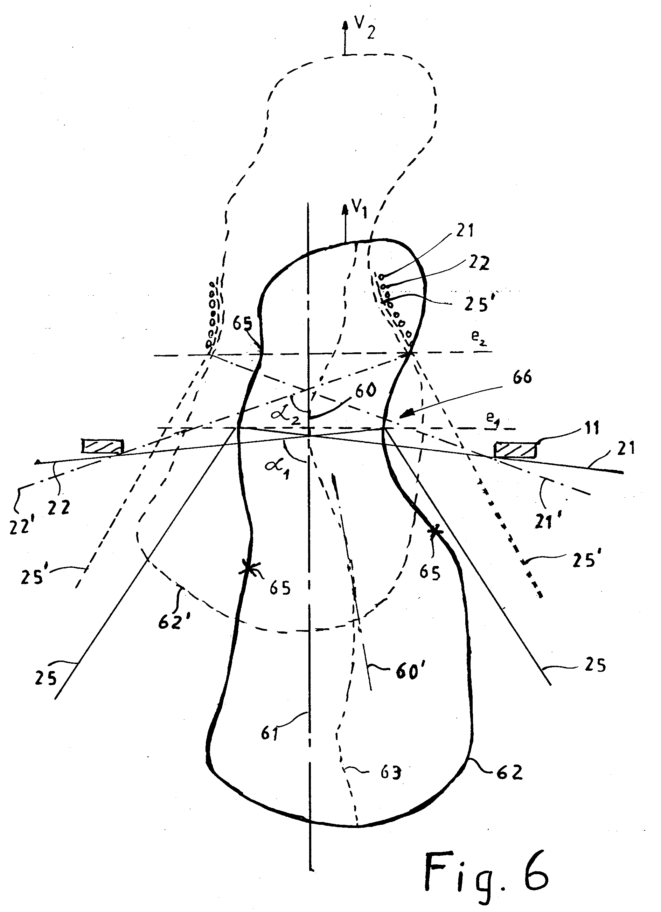

In Fig. 6 sind zwei Positionen eines unregelmäßigen Stützkernes 62 bzw. 62' gezeigt, bei dem die Lageveränderung des Bauteiles bzw. Stützkernes im Flechtprozeß deutlichwird. Der Stützkern 62 wird in Pfeilrichtung durch das Fadenauge 11 gezogen, und zwar derart, daß der Mittelpunkt, der durch die gestrichelte Linie 63 angedeutet ist, in der Flechtebene e₁ stets auf der Flechtmaschinenachse 61 zu liegen kommt. Die örtliche Bauteilachse 60 verläuft dabei gleichzeitig parallel zur Maschinenachse 61. In der in Fig. 6 mit voller Linie gezeichneten Stützkernposition (62) befindet sich der Flechtbereich auf der Ebene e₁. Der Mittelpunkt und die Bauteilachse 60 dieser Ebene e₁ liegen auf der Maschinenachse 61. Die Kreuze 65 des Stützkernes 62 in der ersten Position deuten den Flechtbereich für die gestrichelt dargestellte zweite Position des Stützkernes 62' an, wobei die zugehörige Bauteilachse 60' zunächst noch außerhalb der Maschinenachse 61 liegt. Wenn die Kreuze 65 die Flechtebene e₂ erreichen, liegt auch die zugehörige momentane Bauteilachse 60' auf der Maschinenachse 61.

Während des Abzugsvorganges wird der Stützkern 62 3-dimensional geschwenkt und gleichzeitig parallel verschoben. Zusätzlich zu dieser Veränderung wird die Orientierung der Flechtfasern 21, 22 durch Variieren der Abzugsgeschwindigkeit v entsprechend der Außenkontur des Stützkernes 62 verändert. Außerdem wird die Fadenspannung der Flechtfasern 21, 22 höher gewählt als die der UD-Fasern 25.

Die einzelnen Flechtfasern 21 bzw. 22 beschreiben im Verlauf des Flechtprozesses eine Schraubenlinie, wie sie in Fig. 3 mit der dickgezeichneten Faser 21 gezeigt ist. Bei diesem Verlauf drückt die Flechtfaser 21 die unidirektionalen Fasern, die der Mantellinie 35 des Stützkernes 10' folgen, an den Stützkern 10 bzw. 10'. Die Steigung der umwickelten Flechtfasern bzw. deren Orientierung am fertigen Bauteil wird durch die geometrischen Verhältnisse der Flechtmaschine 12, dem Querschnitt des Bauteiles bzw. Stützkernes 10 und der Geschwindigkeit, mit der das Bauteil 10 bewegt wird, bestimmt. Durch Automatisierung und Programmierung des Greiferbewegungsmechanismus kann die Abzugsgeschwindigkeit entsprechend der Geometrie bzw. dem Querschnitt des Bauteiles 10 verändert werden, um die gewünschte Orientierung der Flechtfasern 21, 22 über das gesamte Bauteil zu erhalten. Die Abzugsbewegung kann entweder mit dem Greifer 13 oder dem Führungsring 14 durchgeführt werden.

Diese Veränderungen werden ebenfalls durch die in Fig. 6 gezeigten zwei Flechtmomente deutlich. In der dargestellten ersten Position des Stützkernes 62 erreicht der Flechtbereich e₁ eine Talsohle einer konkaven Außenkontur 66. In einem derartigen Bereich wird die UD-Faser 25 sich vom Kern 62 ablösen wollen. Um diese Möglichkeit zu vermeiden, wird der Kern 62 mit einer relativ geringen Abzugsgeschwindigkeit v₁ bewegt, so daß die Flechtfasern fast senkrecht zur Maschinenachse 61 zu liegen kommen, d.h., der Winkel α ist ≧ 80. Die Fasern legen sich fast in Umfangsrichtung um den Stützkern 62 und drücken damit die UD-Fasern 25 an die Stützkernoberfläche. Durch eine etwas höhere Fadenspannung für die Flechtfasern 21, 22 wird ein Abheben der UD-Fasern 25 schließlich vollkommen vermieden. In der gestrichelt dargestellten zweiten Position des Stützkernes 62' ist in der Flechtebene e₂ die Tendenz zum Abheben geringer, so daß mit einer höheren Geschwindigkeit v₂ gefahren werden kann. Der Winkel α₂ zwischenden Flechtfasern 21', 22' und der Maschinenachse 61 verringert sich dabei, so daß α₂ < α₁ ist. In Bereichen mit konstantem Querschnitt kann der Winkel α durch entsprechende Abzugsgeschwindigkeit v so eingestellt werden, daß er den zur Erzielung der mechanischen Eigenschaften des Bauteiles gewünschten Wert einnimmt.

Sowohl die Flechtfasern 21, 22 als auch die unidirektionalen Fasern 25 können vorab mittels Tränkeinrichtungen mit einer Matrix getränkt werden. Die Aushärtung des Harzes wird mittels der über dem Fadenauge 11 vorgesehenen Heizeinrichtung 37 bewirkt. Die Einführung des Matrixmaterials sowie deren Härtung kann auch auf jede andere bekannte Art durchgeführt werden.

Bei den bisher beschriebenen Schritten bilden die unidirektionalen Fasern 25 eine innere Faserschicht, die von dem Flechtwerk 55 umgeben ist. In einem Längsschnitt durch das fertige Bauteil 40, wie in Fig. 4 gezeigt, ist an der Oberfläche 41 des Stützkernes 10 eine Längsfaser 25 zu sehen, auf der sich die Flechtfasern 21 bzw. 22 befinden. In diesem Fall, legt sich die unidirektonale Faser 25 genau parallel an die Mantellinie 35 des Stützkernes 10 an. Sie ist dabei gestreckt, wodurch das Bauteil die optimale Festigkeit für Druck/Zug-Belastungen hat.

Bei Bauteilen mit starken 3-dimensionalen Krümmungen kann zusätzlich zu den vorstehend beschriebenen Maßnahmen zumindest ein Teil der unidirektionalen Fasern 25 innerhalb der Flechtfasern eine tangentiale Führung erhalten, um ein seitliches Abrutschen oder Verschieben der unter Zugspannung stehenden UD-Fasern 25 in den Krümmungsbereichen 38 des Stützkernes 10 zu vermeiden. In diesem Fall wird der eine Teil der unidirektionalen Fasern 25 nicht innerhalb des Flechtringes, sondern durch den Flechtring 14 geführt. Dieses ist in Fig. 2 näher gezeigt, bei der der innere Ring 50 nicht alle, wie oben beschrieben, sondern nur einen Großteil der unidirektionalen Fasern 25 führt, während im Führungsring 14 vorgesehene Fadenaugen 51 für unidirektionale Fasern 25'' vorgesehen sind. Diese Fasern 25'' werden abwechselnd von der einen Klöppelgruppe 17 unterfahren und von der anderen Klöppelgruppe 19 überfahren, so daß diese unidirektionalen Fasern 25'' zusätzlich zur radialen Führung durch die Flechtfasern 21, 22 auch eine tangentiale Führung erfahren, wie es in Fig. 3 mit den Pfeilen 52 angedeutet ist. Damit werden sie in ihrer Position, d.h. entlang der entsprechenden Mantellinie 35 des Stützkernes 10' gehalten.

In diesem Fall werden, wie Fig. 5 zeigt, die entsprechenden unidirektionalen Fasern 25'' stellen weise durch Flechtfasern 21 unterlegt und dazwischen mit Flechtfasern 22 umwickelt, so daß die unidirektionale Faser 25'' ihre optimale gespannte Lage verliert. Mit sehr dünnen Flechtfasern 21 bzw. 22 kann dieser Nachteil minimiert werden, wenn es darauf ankommt. Das Verhältnis der durch den inneren Ring 50 und den Führungsring 14 geführten unidirektionalen Fasern 25 bzw. 25'' kann beliebig gewählt werden. Das Verhältnis wird sich in der Regel nach dem jeweiligen Anwendungsfall und dessen Anforderungen richten.

Für die Herstellung eines Schaftes für ein Hüftgelenk-Implantat mit unidirektionalen Fasern wird man das Verhältnis zwischen im Innenring 50 und im Führungsring 14 geführten unidirektionalen Fasern 25 bzw. 25'' möglichst groß wählen.

## Patentansprüche

1. Verfahren zur Herstellung von Bauteilen mit mindestens einer Lage aus längs des Bauteiles gerichteten unidirektionalen Fasern, bei dem ein vorgefertigter Stützkern zusammen mit das Bauteil zumindest teilweise umgebenden, unter Spannung gehaltenen, unidirektionalen Fasern durch eine Flechtmaschine geführt wird, wobei die unidirektionalen Fasern sowie ein Flechtwerk auf das Bauteil aufgelegt und mittels Harz verklebt werden, dadurch gekennzeichnet, daß im Prozeß des Auflegens der UD-Fasern (25, 25', 25'') und der Flechtfasern (21, 22) die Flechtfasern unter höherer Fadenspannung gehalten werden als die UD-Fasern und daß das Bauteil (10, 62) während des Prozesses so bewegt wird, daß die Bauteilachse (60, 60') des jeweiligen Flechtbereiches (e₁, e₂) annähernd auf der Achse (61) der Flechtmaschine (12) liegt.

2. Verfahren nach Anspruch 1, dadurch gekennzeichnet, daß die Abzugsgeschwindigkeit (v) zur Anpassung des Flechtfaserwinkels α an die Bauteiloberfläche variiert wird.

3. Verfahren nach Anspruch 2, dadurch gekennzeichnet, daß die Flechtfasern (21, 22) in Bereichen des Bauteiles mit sich veränderndem Querschnitt einen größeren Wirbel (α₁) mit der Maschinenachse (61) bilden.

4. Verfahren nach einem der Ansprüche 1 bis 3, dadurch gekennzeichnet, daß mehrere miteinander verbundene Stützkerne (10, 62) in einem Arbeitsgang mit UD- und Flechtfasern (25 bzw. 21, 22) überzogen werden.

5. Verfahren nach einem der Ansprüche 1 bis 3, dadurch gekennnzeichnet, daß in einem Arbeitsgang ohne Unterbrechung der UD- und Flechtfasern (25 bzw. 21, 22) Bauteile serienmäßig durch maschinelles Einführen von Stützkernen (10, 62) in die Flechtmaschine (12) hergestellt werden.

6. Verfahren nach einem der vorhergehenden Ansprüche, dadurch gekennzeichnet, daß die unidirektionalen Fasern (25) zwischen dem Stützkern (10, 10') und den Flechtfasern (21, 22) geführt werden.

7. Verfahren nach einem der vorhergehenden Ansprüche, dadurch gekennzeichnet, daß die unidirektionalen Fasern (25, 25'') zumindest teilweise auf der Ebene der Flechtfasern (21, 22) geführt werden, derart, daß sie umflochten werden.

8. Verfahren nach Anspruch 7, dadurch gekennzeichnet, daß sehr dünne Flechtfasern (21, 22) verwendet werden.

9. Verfahren nach einem der vorhergehenden Ansprüche, gekennzeichnet durch Anwendung des Verfahrens zur Herstellung von Knochenimplantaten.
